(19) Europäisches Patentamt
European Patent Office
Office européen des brevets

(11) Veröffentlichungsnummer: **0 123 095**
**B1**

(12) **EUROPÄISCHE PATENTSCHRIFT**

(45) Veröffentlichungstag der Patentschrift: **26.10.88**

(21) Anmeldenummer: **84102659.4**

(22) Anmeldetag: **12.03.84**

(51) Int. Cl.⁴: **C 07 D 491/04**, C 07 F 7/10, A 61 K 31/495 // C07C101/28, C07D307/66 ,(C07D491/04, 307:00, 221:00)

(54) **Chromon- und thiochromonsubstituierte 1,4-Dihydropyridinlactone, mehrere Verfahren zu ihrer Herstellung und ihre Verwendung in Arzneimitteln.**

(30) Priorität: **25.03.83 DE 3311003**

(43) Veröffentlichungstag der Anmeldung: **31.10.84 Patentblatt 84/44**

(45) Bekanntmachung des Hinweises auf die Patenterteilung: **26.10.88 Patentblatt 88/43**

(84) Benannte Vertragsstaaten: **AT BE CH DE FR GB IT LI LU NL SE**

(56) Entgegenhaltungen:
**EP - A - 0 071 819**
**EP - A - 0 080 220**
**DE - A - 3 315 805**
**DE - A - 3 339 861**

**Die Akte enthält technische Angaben, die nach dem Eingang der Anmeldung eingereicht wurden und die nicht in dieser Patentschrift enthalten sind.**

(73) Patentinhaber: **BAYER AG, Konzernverwaltung RP Patentabteilung, D-5090 Leverkusen 1 Bayerwerk (DE)**

(72) Erfinder: **Goldmann, Siegfried, Dr., Kuckuckstrasse 41, D-5600 Wuppertal 2 (DE)**
Erfinder: **Bossert, Friedrich, Dr., Claudiusweg 7, D-5600 Wuppertal 1 (DE)**
Erfinder: **Schramm, Matthias, Dr., Paffrather Strasse 38, D-5000 Köln 80 (DE)**
Erfinder: **Thomas, Günter, Dr., Claudiusweg 9, D-5600 Wuppertal 1 (DE)**
Erfinder: **Gross, Rainer, Dr., Platzhoffstrasse 23, D-5600 Wuppertal 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die vorliegende Erfindung betrifft neue 1,4-Dihydropyridine, mehrere Verfahren zu ihrer Herstellung sowie ihre Verwendung in Arzneimitteln, insbesondere in kreislaufbeeinflussenden Arzneimitteln.

Aus EP-A 71 819 sind bereits Dihydropyridine bekannt, die sich jedoch von den erfindungsgemässen Dihydropyridinen durch die Substitution im Phenylring unterscheiden.

Die neuen Dihydropyridine sind durch folgende allgemeine Formel (I) gekennzeichnet:

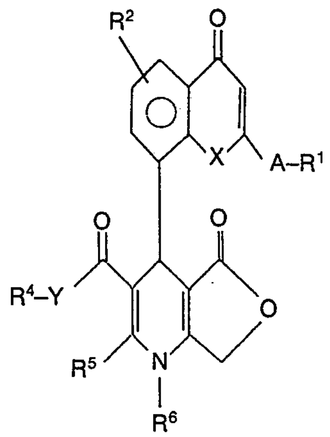

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen, in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle bzw. Heteroaryle gegebenenfalls einen bis 5 gleiche oder verschiedene Substituenten aus der Gruppe Fluor, Chlor, Brom, Jod, $C_1$–$C_8$ Alkyl, $C_1$–$C_8$ Alkylthio, $C_1$–$C_8$ Alkylsulfinyl, Cyano, Hydroxy, Nitro, Mono- oder Polyfluor-$C_1$–$C_4$-alkyl, Mono- oder Polyfluoralkoxy mit 1 bis 4 C-Atomen, Mono- oder Polyfluoralkylthio mit 1 bis 4 C-Atomen, Amino, Monoalkylamino mit 1 bis 5 C-Atomen, Dialkylamino mit jeweils 1 bis 5 C-Atomen aufweisen,

$R^2$ für ein bis drei Halogenatome oder Wasserstoff steht und

$R^4$ für einen geradkettigen, verzweigten oder cyclischen, gesättigten oder ungesättigten Kohlenwasserstoffrest mit 1 bis 18 C-Atomen steht, der gegebenenfalls substituiert ist durch $C_1$–$C_8$ Alkoxy, $C_1$–$C_8$-Alkylthio, $C_1$–$C_8$ Alkylsulfinyl, Trialkylsilyl mit jeweils 1 bis 5 C-Atomen, Cl, Br, J, F, Cyano, Hydroxy, Amino, Alkylamino mit 1 bis 5 C-Atomen, Dialkylamino mit jeweils 1 bis 5 C-Atomen, Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, $C_1$–$C_5$ Alkyl, $C_1$–$C_5$ Alkoxy, $C_1$–$C_4$-Alkylthio, $C_1$–$C_4$ Alkylsulfinyl,

$R^5$ für Wasserstoff oder einen geradkettigen, verzweigten oder cyclischen, gesättigten oder ungesättigten aliphatischen $C_1$–$C_8$ Alkylrest steht, der gegebenenfalls ein oder zwei gleiche oder verschiedene Heterokettenglieder als der Gruppe O, CO, S oder N-Alkyl mit 1 bis 6 C-Atomen enthält und der gegebenenfalls substituiert ist durch Halogen, Nitro, Cyano, Hydroxy, Amino, Mono-$C_1$–$C_5$-alkylamino oder Dialkylamino mit jeweils 1 bis 5 C-Atomen,

$R^6$ für Wasserstoff oder einen geradkettigen oder verzweigten $C_1$–$C_{20}$ Alkylrest steht, der gegebenenfalls substituiert ist durch $C_1$–$C_{10}$ Alkoxy, Halogen oder Morpholino,

A für eine einfache Bindung, eine $C_1$–$C_{20}$ Alkylenkette, die gegebenenfalls durch O oder S unterbrochen sein kann, mit der Voraussetzung, dass wenn A für Einfachbindung steht, der Rest $R^1$ nicht Wasserstoff sein kann,

X für O oder S steht und

Y für eine einfache Bindung, O, S, –NH– oder –N–Alkyl mit 1 bis 8 C-Atomen steht,

in Form von Isomeren, Isomerengemischen, Racematen und optischen Antipoden sowie ihren pharmazeutisch unbedenklichen Salzen.

Als Salze seien beispielsweise genannt Hydrochloride, Hydrogensulfate, Sulfate, Hydrogenphosphate, Acetate, Maleate, Benzoate, Citronate, Tartrate oder Lactate.

Von besonderem Interesse sind Verbindungen der allgemeinen Formel (I), in welcher

$R^1$ für Wasserstoff, einen geradkettigen, cyclischen oder verzweigten aliphatischen Kohlenwasserstoff mit 1 bis 7 C-Atomen, einen Carbonsäurealkylester mit 1 bis 6 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, Benzimidazolyl oder Chinazolyl steht, wobei die genannten Aryle bzw. Heteroaryle gegebenenfalls einen bis 4 gleiche oder verschiedene Substituenten aus der Gruppe Fluor, Chlor, Brom, $C_1$–$C_6$-Alkyl, $C_1$–$C_6$-Alkylthio, $C_1$–$C_6$-Alkylsulfinyl, Cyano, Hydroxy, Nitro, Mono- oder Polyfluoralkyl mit 1 bis 3 C-Atomen, Mono- oder Polyfluoralkoxy mit 1 bis 3 C-Atomen, Amino, Monoalkylamino mit 1 bis 4 C-Atomen, Dialkylamino mit jeweils 1 bis 4 C-Atomen aufweisen,

$R^2$ für Wasserstoff oder ein bis drei Fluoratome steht,

$R^4$ für einen geradkettigen, verzweigten oder cyclischen, gesättigten oder ungesättigten Kohlenwasserstoffrest mit 1 bis 12 C-Atomen steht, der gegebenenfalls substituiert ist durch $C_1$–$C_6$ Alkoxy, $C_1$–$C_6$ Alkylthio, $C_1$–$C_6$ Alkylsulfinyl, Trialkylsilyl mit jeweils 1 bis 3 C-Atomen, Cl, Br, F, Cyano, Hydroxy, Amino, Alkylamino mit 1 bis 4 C-Atomen, Dialkylamino mit jeweils 1 bis 4 C-Atomen, Morpholinyl, Piperidyl, Piperazinyl, Nitro, Nitrat, Phenyl, Naphthyl, Thienyl, Furyl, Pyrryl, Imidazolyl, Oxazolyl, Thiazolyl, Pyridyl, Pyrimidyl,

Pyrazinyl, Indolyl, wobei die genannten Aromaten oder Heteroaromaten gegebenenfalls substituiert sein können durch 1 bis 3 gleiche oder verschiedene Substituenten aus der Gruppe F, Cl, Br, $C_1$–$C_4$ Alkyl, $C_1$–$C_4$ Alkoxy, $C_1$–$C_3$ Alkylthio, $C_1$–$C_3$ Alkylsulfinyl,

$R^5$ für Wasserstoff oder einen geradkettigen, verzweigten oder cyclischen, gesättigten oder ungesättigten aliphatischen Alkylrest mit 1 bis 6 C-Atomen steht, der gegebenenfalls ein oder zwei gleiche oder verschiedene Heterokettenglieder aus der Gruppe O, CO, S oder N-Alkyl mit 1 bis 4 C-Atomen enthält und der gegebenenfalls substituiert ist durch Halogen, Nitro, Cyano, Hydroxy, Amino, Mono-$C_1$–$C_4$-alkylamino oder Dialkylamino mit jeweils 1 bis 4 C-Atomen,

$R^6$ für Wasserstoff oder einen geradkettigen oder verzweigten $C_1$–$C_{12}$ Alkylrest steht, der gegebenenfalls substituiert ist durch $C_1$–$C_6$ Alkoxy, Halogen oder Morpholino,

A für eine einfache Bindung, eine $C_1$–$C_{16}$ Alkylenkette, die gegebenenfalls durch O oder S unterbrochen sein kann, mit der Voraussetzung, dass wenn A für Einfachbindung steht, der Rest $R^1$ nicht Wasserstoff sein kann,

X für O oder S steht und

Y für eine einfache Bindung, O, S, –NH– oder –N(Alkyl)- mit 1 bis 4 C-Atomen steht.

Die erfindungsgemässen Verbindungen der allgemeinen Formel (I) können hergestellt werden, indem man

A) Aldehyde der allgemeinen Formel (II)

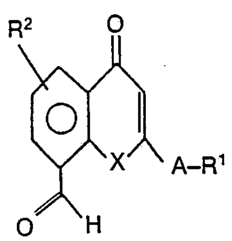

(II)

in welcher

$R^1$, $R^2$, A und X die oben angegebene Bedeutung haben

mit Enaminen der allgemeinen Formel (III)

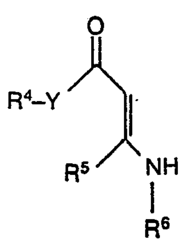

(III)

in welcher

$R^4$, $R^5$, $R^6$ und Y die oben angegebene Bedeutung haben und

Ketonen der allgemeinen Formel (IV)

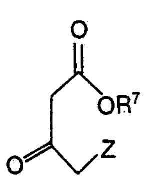

(IV),

in welcher

$R^7$ eine Alkylkette mit 1 bis 6 C-Atomen darstellt und

Z für Halogen oder –O–B steht, wobei B für eine Alkoholschutzgruppe wie

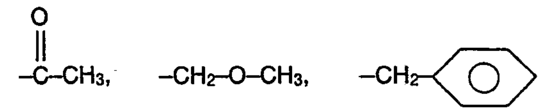

–Si(Alkyl)$_3$ steht

oder

gegebenenfalls in Gegenwart inerter organischer Lösungsmittel bei Temperaturen zwischen 20 und 150 °C umsetzt und im Falle von Z = –O–B die Schutzgruppe mit geeigneten Agentien entfernt

oder

B) Aldehyde der allgemeinen Formel (II) in welcher $R^1$, $R^2$, A und X die oben angegebene Bedeutung haben mit Ketonen der allgemeinen Formel (V)

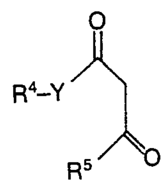

(V)

in welcher

$R^4$ und $R^5$ die oben angegebene Bedeutung haben und

Enaminen der allgemeinen Formel (VI)

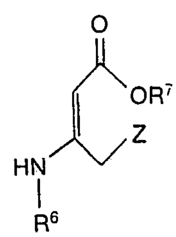

(VI)

in welcher

$R^6$, $R^7$ und Z die oben angegebene Bedeutung haben,

gegebenenfalls in Gegenwart inerter organischer Lösungsmittel bei Temperaturen zwischen 20 und 150 °C umsetzt und im Falle von Z = O–B, worin B die oben angegebene Bedeutung hat, anschliessend die Schutzgruppe mit geeigneten Agentien entfernt

oder

C) Ylidenverbindungen der allgemeinen Formel (VII)

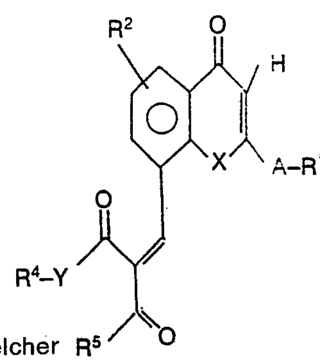

(VII)

in welcher

$R^1$, $R^2$, $R^4$, $R^5$, A, X und Y die oben angegebene Bedeutung haben,

mit Enaminen der allgemeinen Formel (VI) in welcher $R^6$, $R^7$ und Z die oben angegebene Bedeutung haben, gegebenenfalls in Gegenwart inerter organischer Lösungsmittel bei Temperaturen zwischen 20 und 150 °C umsetzt und im Falle von Z = O–B, worin B die oben angegebene Bedeutung hat, die Schutzgruppe anschliessend mit geeigneten Agentien abspaltet,

oder

D) Aldehyde der allgemeinen Formel (II) in welcher $R^1$, $R^2$, A und X die oben angegebene Bedeutung haben, mit Ketonen der allgemeinen Formel (V) in welcher $R^4$, $R^5$ und Y die oben angegebene Bedeutung haben und mit Tetronsäureamiden der allgemeinen Formel (VIII)

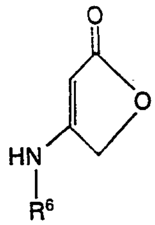

(VIII)

in der

$R^6$ die oben angegebene Bedeutung hat,

gegebenenfalls in Gegenwart inerter organischer Lösungsmittel bei Temperaturen zwischen 20° und 150 °C umsetzt

oder

E) Benzylidenverbindungen der allgemeinen Formel (IX)

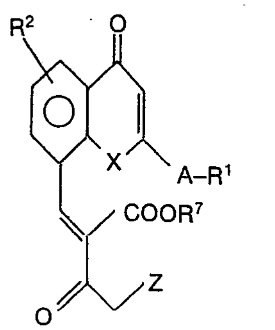

(IX)

in welcher

$R^1$, $R^2$, $R^7$, A, X und Z die oben angegebene Bedeutung haben,

mit Enaminen der allgemeinen Formel (III), in welcher $R^4$, $R^5$, $R^6$ und Y die oben angegebene Bedeutung haben, gegebenenfalls in inerten organischen Lösungsmitteln bei Temperaturen von 20° bis 150 °C umsetzt und im Falle von Z = O–B, worin B die oben angegebene Bedeutung hat, die Schutzgruppe mit geeigneten Agentien abspaltet.

Als geeignete Verfahren zur Abspaltung der Schutzgruppe B in den Verfahren A, B, C und E seien beispielsweise genannt:

Sauer katalysierte Abspaltung (z.B. im Falle von

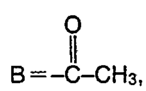

$-CH_2-O-CH_3$),

die basische Abspaltung (z.B. im Falle von

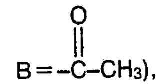

die hydrogenolytische Abspaltung (z.B. im Falle von

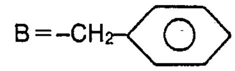

oder die Abspaltung mit Fluorid (z.B. im Falle von B = Si-$(Alkyl)_3$).

Die Abspaltung kann bei Temperaturen von 0 bis 150 °C durchgeführt werden.

Die Verfahrensvarianten A, C und E sind bevorzugt.

Das Mengenverhältnis der Reaktanden zueinander ist beliebig, bevorzugt werden äquimolare Mengen eingesetzt. Die Verbindungen der Formeln (III), (V), (VI) können jedoch auch im Überschuss bis zu 3 Molen eingesetzt werden.

Die Umsetzungstemperaturen aller Verfahrensvarianten betragen bevorzugt 30 bis 120 °C, insbesondere die Siedetemperaturen der benutzten Lösungsmittel.

Wenn in Gegenwart von organischen Lösungsmitteln gearbeitet wird, kommen alle inerten Lösungsmittel in Betracht, wie beispielsweise Alkohole, Essigsäure, Benzol und/oder Toluol.

Die zur Herstellung verwendeten Aldehyde der Formel (II) sind neu und können hergestellt werden, indem man im Falle X = S Thiochromone der Formel

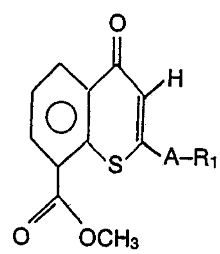

in der

$R_1$, $R_2$ und A die bereits genannten Bedeutungen besitzen,

zu Benzylalkoholen reduziert und

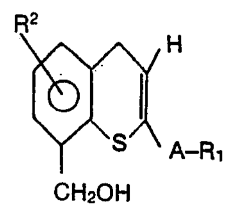

mit Oxidationsmitteln zu Aldehyden oxidiert.

Die als Ausgangsstoffe verwendeten Thiochromone sind bekannt oder können nach bekannten Verfahren hergestellt werden (Bossert, Lieb. Ann. 680, 40 (1964)).

Für die Reduktion zum Benzylalkohol können inerte organische Lösungsmittel eingesetzt werden, beispielsweise Ether wie z.B. Dioxan, Diethylether, Tetrahydrofuran, Dimethoxyethan oder Aromaten wie z.B. Toluol oder Benzol. Als Reduktionsmittel seien beispielhaft Alkalialuminiumhydride wie z.B. $LiAlH_4$ oder Alkylaluminium-

hydride wie z.B. Diisobutylaluminiumhydrid erwähnt.

Bei der Durchführung dieses Verfahrens arbeitet man vorzugsweise in einem Temperaturbereich von −100 °C bis +60 °C, insbesondere in einem Bereich von −60 °C bis +30 °C.

Die Umsetzung erfolgt üblicherweise bei Normaldruck, kann aber auch bei erhöhtem Druck durchgeführt werden.

Das Reduktionsmittel wird in dem Fachmann geläufigen Mengen zugesetzt, bevorzugt in mindestens vier und höchstens 8 Hydridäquivalenten.

Für die Oxidation des Benzylalkohols zum Aldehyd können die gleichen Lösungsmittel wie bei der Reduktion Verwendung finden, zusätzlich aber auch halogenierte Kohlenwasserstoffe, wie Chloroform und Methylenchlorid oder Ketone wie z.B. Aceton.

Als Oxidationsmittel können die üblicherweise für Oxidationen eingesetzten Übergangsmetalloxide eingesetzt werden, vorzugsweise jedoch Mangandioxid.

Bei der Durchführung der Oxidation arbeitet man normalerweise in einem Temperaturbereich von −30 bis +200 °C, vorzugsweise beim Siedepunkt des jeweiligen Lösungsmittels. Die Oxidation erfolgt üblicherweise bei Normaldruck, kann aber auch bei erhöhtem Druck durchgeführt werden.

Das Oxidationsmittel kann in 3 bis 20, vorzugsweise in 5 bis 10 Oxidationsäquivalenten eingesetzt werden. Auch kann es zweckmässig sein, von Zeit zu Zeit frisches Oxidationsmittel zum Reaktionsansatz zuzugeben.

Im Falle X = O werden Chromone der Formel (X)

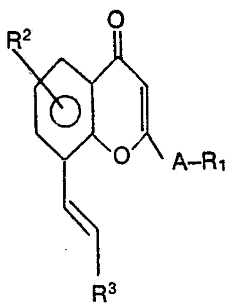

(X)

in der

$R^1$, $R^2$ und Y die oben angegebene Bedeutung haben, mit der Einschränkung, dass A keine Alkenylenkette ist oder Schwefel enthält und

$R^3$ für Wasserstoff oder Alkyl (mit 1 bis 10 C-Atomen) steht, mit Ozon in Anwesenheit inerter organischer Lösungsmittel umsetzt, und anschliessend reduktiv aufarbeitet.

Die als Ausgangsstoffe verwendeten 8-Alkenylchromone sind bekannt oder können nach bekannten Verfahren hergestellt werden (US-Patent 3 350 411, vgl. auch Synthesis 1982, 221).

Für die Ozonolyse seien als inerte Lösungsmittel genannt: chlorierte Kohlenwasserstoffe wie z.B. Methylenchlorid, Chloroform oder Tetrachlorkohlenstoff, Ester wie z.B. Essigsäureethylester, Alkohole wie z.B. Methanol oder Ethanol, Säuren wie z.B. Ameisensäure oder Essigsäure.

Die Ozonolyse erfolgt bei −100 °C bis 20 °C, vorzugsweise jedoch bei −80 °C bis −30 °C mit anschliessender reduktiver Aufarbeitung z.B. mit Dimethylsulfid, Zinkstaub, katalytischer Hydrierung oder Natriumdithionit.

Auf ein Mol Chromon (X) wird nur ein Mol Ozon verwendet um eine Spaltung weiterer Doppelbindungen zu verhindern.

Die zur Herstellung verwendeten Enamine der allgemeinen Formel (III) sind bekannt oder können nach bekannten Verfahren hergestellt werden (vgl. A.C. Cope, J. Am. Chem. Soc. 67, 1017 (1945)).

Die zur Herstellung verwendeten β-Ketoester der allgemeinen Formel (IV) sind bekannt oder können nach bekannten Verfahren hergestellt werden (vgl. Gelin, Pallet, Synth. Comm. 1980, 805; Tetrahedron 34, 1453 (1978)). Oder: käuflich bei LONZA AG (Z = Cl).

Die zur Herstellung verwendeten Ketone der Struktur (V) sind bekannt oder können nach bekannten Verfahren hergestellt werden: z.B. für Y=O, D. Borrmann, Umsetzung von Diketen mit Alkoholen, Phenolen und Mercaptanen in Houben-Weyl, Methoden der Organischen Chemie, Vol. VII 14, 230ff. (1968), Y. Dikawa, K. Sugano und O. Yonemitsu, J. Org. Chem. 43, 2087 (1978).

Die zur Herstellung verwendeten β-Aminocrotonsäureester der allgemeinen Formel (VI) können nach bekannten Verfahren hergestellt werden (vgl. A.C. Cope, J. Amer. Chem. Soc. 67, 1017 (1945)).

Die zur Herstellung verwendeten Benzylidenverbindungen der Struktur (VII) sind neu, können aber nach bekannten Verfahren hergestellt werden (vgl. G. Jones, «The Knoevenagel Condensation», in Org. Reactions Vol. XV, 204 ff (1967)).

Die erfindungsgemässen Verbindungen zeigen ein nicht vorhersehbares und wertvolles pharmakologisches Wirkungsspektrum. Sie können als Cardiotonika zur Verbesserung der Herzkontraktilität dienen. Darüber hinaus können sie dadurch, dass sie den $Ca^{++}$-Einstrom in die Zelle erhöhen, als Antihypotonica, zur Senkung von Blutzucker, zur Abschwellung von Schleimhäuten und zur Beeinflussung des Salz- und/oder Flüssigkeitshaushaltes eingesetzt werden.

Die erfindungsgemässen Verbindungen können in bekannter Weise in die üblichen Formulierungen überführt werden, wie Tabletten, Kapseln, Dragees, Pillen, Granulate, Aerosole, Sirupe, Emulsionen, Suspensionen und Lösungen, unter Verwendung inerten, nichttoxischer, pharmazeutisch geeigneter Trägerstoffe oder Lösungsmittel. Hierbei soll die therapeutisch wirksame Verbindung jeweils in einer Konzentration von etwa 0,5 bis 90 Gew.-% der Gesamtmischung vorhanden sein, d.h. in Mengen, die ausreichend sind, um den angegebenen Dosierungsspielraum zu erreichen.

Die Formulierungen werden beispielsweise hergestellt durch Verstrecken der Wirkstoffe mit Lösungsmitteln und/oder Trägerstoffen, gegebenenfalls unter Verwendung von Emulgiermitteln und/oder Dispergiermitteln, wobei z.B. im Fall der Benutzung von Wasser als Verdünnungsmittel gegebenenfalls organische Lösungsmittel als Hilfslösungsmittel verwendet werden können.

Als Hilfsstoffe seien beispielsweise aufgeführt: Wasser, nicht-toxische organische Lösungsmittel, wie Paraffine (z.B. Erdölfraktionen), pflanzliche Öle (z.B. Erdnuss-/Sesamöl), Alkohole (z.B. Ethylalkohol, Glycerin), Glykole (z.B. Propylenglykol, Polyethylenglykol), feste Trägerstoffe, wie z.B. natürliche Gesteinsmehle (z.B. Kaoline, Tonerden, Talkum, Kreide), synthetische Gesteinsmehle (z.B. hochdisperse Kieselsäure, Silikate), Zucker (z.B. Rohr-, Milch- und Traubenzucker), Emulgiermitteln (z.B. Polyoxyethylen-Fettsäure-Ester, Polyoxyethylen-Fettalkohol-Ether, Alkylsulfonate und Arylsulfonate), Dispergiermittel (z.B. Lignin, Sulfitablaugen, Methylcellulose, Stärke und Polyvinylpyrrolidon) und Gleitmittel (z.B. Magnesiumstearat, Talkum, Stearinsäure und Natriumlaurylsulfat).

Die Applikation erfolgt in üblicher Weise, vorzugsweise oral oder parenteral, insbesondere perlingual oder intravenös. Im Falle der oralen Anwendung können Tabletten selbstverständlich ausser den genannten Trägerstoffen auch Zusätze, wie Natriumcitrat, Calciumcarbonat und Dicalciumphosphat zusammen mit verschiedenen Zuschlagstoffen, wie Stärke, vorzugsweise Kartoffelstärke, Gelatine und dergleichen enthalten. Weiterhin können Gleitmittel, wie Magnesiumstearat, Natriumlaurylsulfat und Talkum zum Tablettieren mitverwendet werden. Im Falle wässriger Suspensionen und/oder Elixieren, die für orale Anwendungen gedacht sind, können die Wirkstoffe ausser mit den obengenannten Hilfsstoffen mit verschiedenen Geschmacksaufbesserern oder Farbstoffen versetzt werden.

Für den Fall der parenteralen Anwendung können Lösungen der Wirkstoffe unter Verwendung geeigneter flüssiger Trägermaterialien eingesetzt werden.

Im allgemeinen hat es sich als vorteilhaft erwiesen, bei intravenöser Applikation Mengen von etwa 0,001 bis 1 mg/kg, vorzugsweise etwa 0,01 bis 0,5 mg/kg Körpergewicht zur Erzielung wirksamer Ergebnisse zu verabreichen, und bei oraler Applikation beträgt die Dosierung etwa 0,01 bis 20 mg/kg, vorzugsweise 0,1 bis 10 mg/kg Körpergewicht.

Trotzdem kann es gegebenenfalls erforderlich sein, von den genannten Mengen abzuweichen, und zwar in Abhängigkeit vom Körpergewicht des Versuchstieres bzw. der Art des Applikationsweges, aber auch aufgrund der Tierart und deren individuellem Verhalten gegenüber dem Medikament bzw. der Art von dessen Formulierung und dem Zeitpunkt bzw. Intervall, zu welchem die Verabreichung erfolgt. So kann es in einigen Fällen ausreichend sein, mit weniger als der vorgenannten Mindestmenge auszukommen, während in anderen Fällen die genannte obere Grenze überschritten werden muss. Im Falle der Applikation, grösserer Mengen kann es empfehlenswert sein, diese in mehrere Einzelgaben über den Tag zu verteilen. Für die Applikation in der Humanmedizin ist der gleiche Dosierungsspielraum vorgesehen. Sinngemäss gelten hierbei auch die obigen Ausführungen.

Beispiel 1

2-Methyl-4-(4-oxo-2-phenyl-4H-thiochromen-8-yl)-5-oxo-1,4,5,7-tetrahydrofuro[3,4-b]-pyridin-3-carbonsäureethylester

a) mit γ-Chloracetessigester

Je 10 mmol 4-Oxo-2-phenyl-4H-thiochromen-8-carboxaldehyd, 3-Aminocrotonsäureethylester und γ-Chloracetessigsäuremethylester werden in 30 ml Ethanol über Nacht am Rückfluss gekocht. Nach Abkühlung wird abgesaugt.

FP: 271–273 °C.

b) mit 4-Acetoxyacetessigester:

Ansatz wie unter a) nur mit 4-Acetoxyacetessigsäureethylester anstatt γ-Chloracetessigester. Nach Rückfluss über Nacht wird mit 2 ml gesättigter ethanolischer HCl versetzt und 1 h am Rückfluss gekocht, gekühlt, abgesaugt und aus viel Ethanol umkristallisiert.

FP: 271–273 °C.

Beispiel 2 (Verfahrensvariante B)

2-Methyl-4-[2-(3-chlorphenyl)-4-oxo-4H-chromen-8-yl]-5-oxo-1,4,5,7-tetrahydro-furo[3,4-b]pyridin-3-carbonsäureethylester

Darstellung der Vorstufe:

3-Amino-4-acetoxy-crotonsäureethylester

$$(VI,\ R^6 = H,\ R^7 = -C_2H_5,\ Z = O\underset{\underset{O}{\|}}{C}CH_3)$$

Eine Lösung von 4-Acetoxy-3-keto-buttersäureethylester und katalyt. Mengen p-Toluolsulfonsäure in Toluol wird am Wasserabscheider erhitzt und solange Ammoniak eingeleitet bis die Wasserentstehung beendet ist.

Es wird mit Wasser gewaschen, getrocknet, eingeengt und der Rückstand destilliert (0,2 mm/ 90 °C).

FP: 51 °C.

Je 10 mmol 2-(3-Chlorphenyl)-4-oxo-4H-chromen-8-carboxaldehyd, Acetessigsäureethylester und 3-Amino-4-acetoxycrotonsäureethylester werden über Nacht in 30 ml Ethanol gekocht, anschliessend mit 2 ml ges. ethanolischer HCl versetzt und eine weitere Stunde erhitzt. Es wird einrotiert und kristallisiert.

FP: > 260 °C

MS: 477 (100%), 448, 404, 256, 222.

Beispiel 3 (Verfahrensvariante C)

2-Methyl-4-(2-cyclohexyl-4-oxo-4H-chromen-8-yl)-5-oxo-1,4,5,7-tetrahydrofuro[3,4-b]pyridin-3-carbonsäuremethylester

Je 10 mmol 1-(2-Cyclohexyl-4-oxo-4H-chromen-8-yl)-1-buten-3-oxo-2-carbonsäuremethylester

(VII, $A{-}R^1 =$ 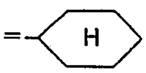 ,

$R^2 = H$, $Y{-}R^4 = OCH_3$, $X = O$, $R^5 = CH_3$) und 3-Amino-4-acetoxycrotonsäureethylester (aus

Beispiel 2) wurden über Nacht in 30 ml Ethanol am Rückfluss gekocht, anschliessend mit 2 ml gesättigter ethanolischer HCl versetzt, 1 h gekocht und eingeengt.
FP: 182–185 °C.

Beispiel 4 (Verfahrensvariante D)
2-Methyl-4-(4-oxo-2-phenyl-4H-thiochromen-8-yl)-5-oxo-1,4,5,7-tetrahydrofuro[3,4-b]pyridin-3-carbonsäuremethylester
Darstellung der Vorstufe:

Tetronsäureamid (VIII, $R^6$ = H)
0,2 Mole 3-Amino-4-acetoxy-crotonsäureethylester (aus Beispiel 2) wurden in 600 ml trockenem Methanol gelöst mit 8 g wasserfreiem $K_2CO_3$ versetzt und 40 min. am Rückfluss gekocht. Nach Abkühlung wurden 10 g $NH_4Cl$ zugegeben, einrotiert und der Rückstand mit Methanol ausgekocht. Aus der Methanollösung kristallisierte Tetronsäureamid aus (FP: 161–163 °C).
Je 10 mmol 1-(4-Oxo-2-phenyl-4H-thiochromen-8-yl)-1-buten-3-oxo-2-carbonsäuremethylester

(VII, A–$R^1$ = 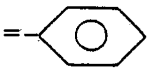 ,

$R^2$ = H, X = S, Y–$R^4$ = $OCH_3$, $R^5$ = $CH_3$) und Tetronsäureamid wurden in 30 ml Ethanol über Nacht am Rückfluss gekocht und chromatographiert.
FP: 270 °C
MS: 445, 443, 238, 224, 208.

Beispiel 5 (Verfahrensvariante E)
2-Methyl-4-(2-octyl-4-oxo-4H-chromen-8-yl)-5-oxo-1,4,5,7-tetrahydrofuro[3,4-b]pyridin-3-carbonsäuremethylester
Je 10 mmol 1-(2-Octyl-4-oxo-4H-chromen-8-yl)-1-buten-4-chlor-3-oxo-3-carbonsäuremethylester und β-Aminocrotonsäuremethylester wurden in 30 ml Ethanol über Nacht am Rückfluss erhitzt, mit 2 ml einer gesättigten ethanolischen HCl versetzt und eine weitere Stunde erhitzt. Es wird gekühlt und eingeengt.
FP: 129–132 °C.

Analog Beispiel 1b wurden dargestellt:

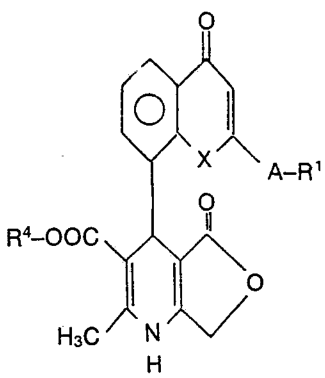

| Beispiel | A–$R^1$ | $R^4$ | X | FP (°C) |
|---|---|---|---|---|
| 6 | 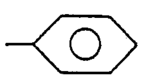 | $-CH_3$ | S | >270 |
| 7 | " | $-(CH_2)_2-CH_3$ | S | 175–80 |
| 8 | " | $-CH_2-CH=CH_2$ | S | 248–51 |
| 9 | " | $-C_4H_9$ | S | >265 |
| 10 | " | $-(CH_2)_7-CH_3$ | S | 114 |
| 11 | " | $-CH_2-$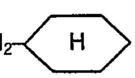 | S | >290 |
| 12 | " | $-CH_2-CH_2-OCH_3$ | S | 268–70 |
| 13 | " | 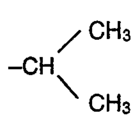 | S | 261–63 |
| 14 | " | 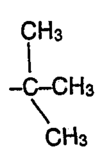 | S | >260 (Zers.) |

| Beispiel | A–R[1] | $R^4$ | X | FP (°C) |
|---|---|---|---|---|
| 15 | 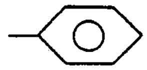 | 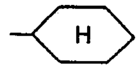 | S | > 260 |
| 16 | " | 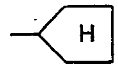 | S | > 260 |
| 17 | " | 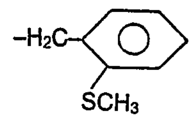 | S | 232–35 |
| 18 | " | 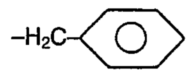 | S | > 275 |
| 19 | " | $-CH_2-CH_2-Si(CH_3)_3$ | S | > 270 |
| 20 | " | $-(CH_2)_2-SCH_3$ | S | > 270 |
| 21 | " | 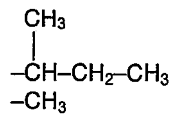 | S | > 300 |
| 22 | " | $-CH_3$ | O | > 260 |
| 23 | 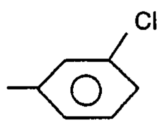 | $-(CH_2)_7-CH_3$ | O | 218–20 |
| 24 | 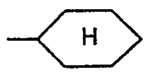 | $-C_2H_5$ | O | 212–15 |
| 25 | " | $-CH_3$ | O | 182–185 |
| 26 | 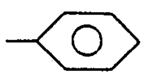 | $-C_2H_5$ | O | > 280 |
| 27 | 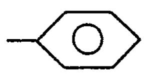 | $-C_4H_9$ | O | > 280 |
| 28 | $-(CH_2)_8-CH_3$ | $-C_2H_5$ | O | 172–175 |
| 29 | 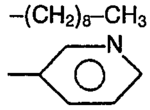 | $-C_2H_5$ | O | > 270 |
| 30 | $-(CH_2)_8-CH_3$ | $-CH_3$ | O | 129–132 |
| 31 | $-C(CH_3)_3$ | $-C_2H_5$ | O | > 270 |
| 32 | $-COOC_2H_5$ | $-C_2H_5$ | O | 140–144 |
| 33 | 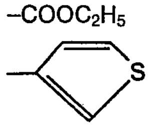 | $-C_2H_5$ | O | > 270 |
| 34 | $-(CH_2)_4-CH_3$ | $-C_2H_5$ | O | 219–221 |
| 35 | $-(CH_2)_4-CH_3$ | $-CH_3$ | O | 222–225 |
| 36 | 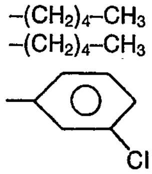 | $-C_2H_5$ | S | > 270 |
| 37 | 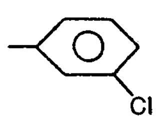 | $-CH_3$ | S | > 270 |

Prüfung der positiv inotropen Wirkung

Versuchsanordnung

Die linken Vorhöfe von Meerschweinchenherzen werden isoliert und in ein thermostatisiertes Organbad gehängt, welches eine isotonische Mineralsalzlösung, die dem Ionenmilieu und dem pH-Wert von Körperflüssigkeiten angepasst ist und geeignete Nährstoffe enthält. Dieses Organbad wird mit einem Gasgemisch bestehend aus Sauerstoff und Kohlendioxyd begast, wobei der

Kohlendioxydgehalt so bemessen ist, dass der pH-Wert des Organbades konstant bleibt. Die linken Vorhöfe werden in das Organbad eingespannt, die Spannung wird mittels eines Kraftaufnehmers registriert, wobei ein bestimmter Grundtonus eingestellt wird. Anschliessend werden die linken Vorhöfe kontinuierlich in bestimmten Abständen elektrisch gereizt und die dabei erfolgenden Kontraktionen registriert. Nach Zugabe des Wirkstoffs werden die Kontraktionen weiterhin registriert. Eine Kontraktionsverstärkung um mindestens 25% gilt als signifikante positiv-inotrope Wirkung.

Hervorgehoben seien solche Verbindungen der allgemeinen Formel (I), die bereits ab einer Konzentration von $10^{-5}$ g/ml am linken Vorhof des isolierten Meerschweinchenherzens in der folgenden Versuchsanordnung eine positiv inotrope Wirkung zeigen.

Beispielhaft seien genannt:

| | Δ dp/dt |
|---|---|
| Beispiel 1 | +55% |
| Beispiel 9 | +42% |
| Beispiel 10 | +41% |
| Beispiel 11 | +57% |

**Patentansprüche**

1. Dihydropyridine der allgemeinen Formel (I)

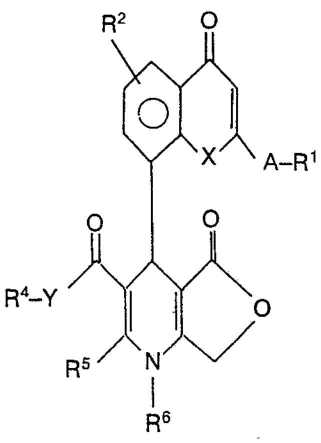

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen, in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle bzw. Heteroaryle gegebenenfalls einen bis 5 gleiche oder verschiedene Substituenten aus der Gruppe Fluor, Chlor, Brom, Jod, $C_1$–$C_8$ Alkyl, $C_1$–$C_8$ Alkylthio, $C_1$–$C_8$ Alkylsulfinyl, Cyano, Hydroxy, Nitro, Mono- oder Polyfluor-$C_1$–$C_4$-alkyl, Mono- oder Polyfluoralkoxy mit 1 bis 4 C-Atomen, Mono- oder Polyfluoralkylthio mit 1 bis 4 C-Atomen, Amino, Monoalkylamino mit 1 bis 5 C-Atomen, Dialkylamino mit jeweils 1 bis 5 C-Atomen aufweisen,

$R^2$ für ein bis drei Halogenatome oder Wasserstoff steht und

$R^4$ für einen geradkettigen, verzweigten oder cyclischen, gesättigten oder ungesättigten Kohlenwasserstoffrest mit 1 bis 18 C-Atomen steht, der gegebenenfalls substituiert ist durch $C_1$–$C_8$ Alkoxy, $C_1$–$C_8$-Alkylthio, $C_1$–$C_8$ Alkylsulfinyl, Trialkylsilyl mit jeweils 1 bis 5 C-Atomen, Cl, Br, J, F, Cyano, Hydroxy, Amino, Alkylamino mit 1 bis 5 C-Atomen, Dialkylamino mit jeweils 1 bis 5 C-Atomen, Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, $C_1$–$C_5$ Alkyl, $C_1$–$C_5$ Alkoxy, $C_1$–$C_4$-Alkylthio, $C_1$–$C_4$ Alkylsulfinyl,

$R^5$ für Wasserstoff oder einen geradkettigen, verzweigten oder cyclischen, gesättigten oder ungesättigten aliphatischen $C_1$–$C_8$ Alkylrest steht, der gegebenenfalls ein oder zwei gleiche oder verschiedene Heterokettenglieder als der Gruppe O, CO, S oder N-Alkyl mit 1 bis 6 C-Atomen enthält und der gegebenenfalls substituiert ist durch Halogen, Nitro, Cyano, Hydroxy, Amino, Mono-$C_1$–$C_5$-alkylamino oder Dialkylamino mit jeweils 1 bis 5 C-Atomen,

$R^6$ für Wasserstoff oder einen geradkettigen oder verzweigten $C_1$–$C_{20}$ Alkylrest steht, der gegebenenfalls substituiert ist durch $C_1$–$C_{10}$ Alkoxy, Halogen oder Morpholino,

A für eine einfache Bindung, eine $C_1$–$C_{20}$ Alkylenkette steht, wobei die Kette gegebenenfalls durch O oder S unterbrochen sein kann, mit der Voraussetzung, dass wenn A für eine Einfachbindung steht, der Rest $R^1$ nicht Wasserstoff sein kann,

X für O oder S steht und

Y für eine einfache Bindung, O, S, –NH– oder –N-Alkyl mit 1 bis 8 C-Atomen steht,

in Form von Isomeren, Isomerengemischen, Racematen und optischen Antipoden sowie ihren pharmazeutisch unbedenklichen Salzen.

2. Verbindungen gemäss Anspruch 1, in denen

$R^1$ für Wasserstoff, einen geradkettigen, cyclischen oder verzweigten aliphatischen Kohlenwasserstoff mit 1 bis 7 C-Atomen, einen Carbonsäurealkylester mit 1 bis 6 C-Atomen in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl, benzimidazolyl oder Chinazolyl steht, wobei die genannten Aryle bzw. Heteroaryle gegebenenfalls einen bis 4 gleiche oder verschiedene Substituenten aus der Gruppe Fluor, Chlor, Brom, $C_1$–$C_6$-Alkyl, $C_1$–$C_6$-Alkylthio, $C_1$–$C_6$-Alkylsulfinyl, Cyano, Hydroxy, Nitro, Mono- oder Polyfluoralkyl mit 1 bis 3 C-Atomen, Mono- oder

Polyfluoralkoxy mit 1 bis 3 C-Atomen, Amino, Monoalkylamino mit 1 bis 4 C-Atomen, Dialkylamino mit jeweils 1 bis 4 C-Atomen aufweisen,

$R^2$ für Wasserstoff oder ein bis drei Fluoratome steht,

$R^4$ für einen geradkettigen, verzweigten oder cyclischen, gesättigten oder ungesättigten Kohlenwasserstoffrest mit 1 bis 12 C-Atomen steht, der gegebenenfalls substituiert ist durch $C_1$–$C_6$ Alkoxy, $C_1$–$C_6$ Alkylthio, $C_1$–$C_6$ Alkylsulfinyl, Trialkylsilyl mit jeweils 1 bis 3 C-Atomen, Cl, Br, F, Cyano, Hydroxy, Amino, Alkylamino mit 1 bis 4 C-Atomen, Dialkylamino mit jeweils 1 bis 4 C-Atomen, Morpholinyl, Piperidyl, Piperazinyl, Nitro, Nitrat, Phenyl, Naphthyl, Thienyl, Furyl, Pyrryl, Imidazolyl, Oxazolyl, Thiazolyl, Pyridyl, Pyrimidyl, Pyrazinyl, Indolyl, wobei die genannten Aromaten oder Heteroaromaten gegebenenfalls substituiert sein können durch 1 bis 3 gleiche oder verschiedene Substituenten aus der Gruppe F, Cl, Br, $C_1$–$C_4$ Alkyl, $C_1$–$C_4$ Alkoxy, $C_1$–$C_3$ Alkylthio, $C_1$–$C_3$ Alkylsulfinyl,

$R^5$ für Wasserstoff oder einen geradkettigen, verzweigten oder cyclischen, gesättigten oder ungesättigten aliphatischen Alkylrest mit 1 bis 6 C-Atomen steht, der gegebenenfalls ein oder zwei gleiche oder verschiedene Heterokettenglieder aus der Gruppe O, CO, S oder N-Alkyl mit 1 bis 4 C-Atomen enthält und der gegebenenfalls substituiert ist durch Halogen, Nitro, Cyano, Hydroxy, Amino, Mono-$C_1$–$C_4$-alkylamino oder Dialkylamino mit jeweils 1 bis 4 C-Atomen,

$R^6$ für Wasserstoff oder einen geradkettigen oder verzweigten $C_1$–$C_{12}$ Alkylrest steht, der gegebenenfalls substituiert ist durch $C_1$–$C_6$ Alkoxy, Halogen oder Morpholino,

A für eine einfache Bindung, eine $C_1$–$C_{16}$ Alkylenkette steht, wobei die Kette gegebenenfalls durch O oder S unterbrochen sein kann, mit der Voraussetzung, dass wenn A für eine Einfachbindung steht, der Rest $R^1$ nicht Wasserstoff sein kann.

X für O oder S steht und

Y für eine einfache Bindung, O, S, –NH– oder –N(Alkyl)– mit 1 bis 4 C-Atomen steht.

3. Verbindungen gemäss Ansprüche 1 bis 2 zur Bekämpfung von Erkrankungen.

4. Verbindungen gemäss Ansprüche 1 bis 2 zur Verbesserung der Herzkontraktilität, als Antihypotonica, zur Senkung von Blutzucker, zur Abschwellung von Schleimhäuten, zur Beeinflussung des Salz- und/oder Flüssigkeitshaushaltes.

5. Verfahren zur Herstellung von Dihydropyridinen der allgemeinen Formel (I)

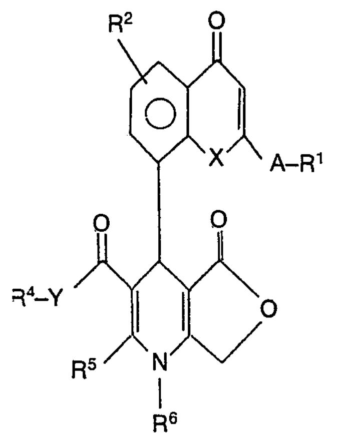

(I)

in welcher

$R^1$ für Wasserstoff, einen geradkettigen, cyclischen oder verzweigten aliphatischen Kohlenwasserstoff mit 1 bis 8 C-Atomen, einen Carbonsäurealkylester mit 1 bis 8 C-Atomen, in der Alkylkette, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Isochinolyl, Indolyl, Benzimidazolyl, Chinazolyl oder Chinoxalyl steht, wobei die genannten Aryle bzw. Heteroaryle gegebenenfalls einen bis 5 gleiche oder verschiedene Substituenten aus der Gruppe Fluor, Chlor, Brom, Jod, $C_1$–$C_8$ Alkyl, $C_1$–$C_8$ Alkylthio, $C_1$–$C_8$ Alkylsulfinyl, Cyano, Hydroxy, Nitro, Mono- oder Polyfluor-$C_1$–$C_4$-alkyl, Mono- oder Polyfluoralkoxy mit 1 bis 4 C-Atomen, Mono- oder Polyfluoralkylthio mit 1 bis 4 C-Atomen, Amino, Monoalkylamino mit 1 bis 5 C-Atomen, Dialkylamino mit jeweils 1 bis 5 C-Atomen aufweisen,

$R^2$ für ein bis drei Halogenatome oder Wasserstoff steht und

$R^4$ für einen geradkettigen, verzweigten oder cyclischen, gesättigten oder ungesättigten Kohlenwasserstoffrest mit 1 bis 18 C-Atomen steht, der gegebenenfalls substituiert ist durch $C_1$–$C_8$ Alkoxy, $C_1$–$C_8$-Alkylthio, $C_1$–$C_8$ Alkylsulfinyl, Trialkylsilyl mit jeweils 1 bis 5 C-Atomen, Cl, Br, J, F, Cyano, Hydroxy, Amino, Alkylamino mit 1 bis 5 C-Atomen, Dialkylamino mit jeweils 1 bis 5 C-Atomen, Morpholinyl, Piperidyl, Piperazinyl, Nitro, Nitrat, Phenyl, Naphthyl, Thienyl, Furyl, Pyrryl, Pyrazolyl, Imidazolyl, Oxazolyl, Isoxazolyl, Thiazolyl, Pyridyl, Pyridazinyl, Pyrimidyl, Pyrazinyl, Chinolyl, Indolyl oder Chinazolyl, wobei die genannten Aromaten oder Heteroaromaten gegebenenfalls substituiert sein können durch 1 bis 3 gleiche oder verschiedene Substituenten aus der Gruppe F, Cl, Br, $C_1$–$C_5$ Alkyl, $C_1$–$C_5$ Alkoxy, $C_1$–$C_4$-Alkylthio, $C_1$–$C_4$ Alkylsulfinyl,

$R^5$ für Wasserstoff oder einen geradkettigen, verzweigten oder cyclischen, gesättigten oder ungesättigten aliphatischen $C_1$–$C_8$ Alkylrest steht,

der gegebenenfalls ein oder zwei gleiche oder verschiedene Heterokettenglieder als der Gruppe O, CO, S oder N-Alkyl mit 1 bis 6 C-Atomen enthält und der gegebenenfalls substituiert ist durch Halogen, Nitro, Cyano, Hydroxy, Amino, Mono-$C_1$–$C_5$-alkylamino oder Dialkylamino mit jeweils 1 bis 5 C-Atomen,

$R^6$ für Wasserstoff oder einen geradkettigen oder verzweigten $C_1$–$C_{20}$ Alkylrest steht, der gegebenenfalls substituiert ist durch $C_1$–$C_{10}$ Alkoxy, Halogen oder Morpholino,

A für eine einfache Bindung, eine $C_1$–$C_{20}$ Alkylenkette steht, wobei die Kette gegebenenfalls durch O oder S unterbrochen sein kann, mit der Voraussetzung, dass wenn A für eine Einfachbindung steht, der Rest $R^1$ nicht Wasserstoff sein kann.

X für O oder S steht und

Y für eine einfache Bindung, O, S, –NH– oder –N-Alkyl mit 1 bis 8 C-Atomen steht,

dadurch gekennzeichnet, dass man

A) Aldehyde der allgemeinen Formel (II)

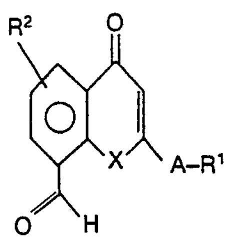

(II)

in welcher

$R^1$, $R^2$, A und X die in den Ansprüchen 1 bis 3 angegebene Bedeutung haben

mit Enaminen der allgemeinen Formel (III)

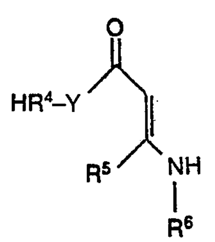

(III)

in welcher

$R^4$, $R^5$, $R^6$ und Y die in den Ansprüchen 1 bis 3 angegebene Bedeutung haben und

Ketonen der allgemeinen Formel (IV)

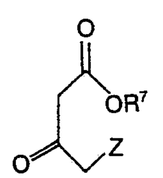

(IV),

in welcher

$R^7$ eine Alkylkette mit 1 bis 6 C-Atomen darstellt und

Z für Halogen oder –O–B steht, wobei B für eine Alkoholschutzgruppe wie

$-\overset{O}{\overset{\|}{C}}-CH_3$, $-CH_2-O-CH_3$, 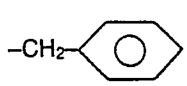

oder $-Si(Alkyl)_3$ steht,

gegebenenfalls in Gegenwart inerter organischer Lösungsmittel bei Temperaturen zwischen 20 und 150 °C umsetzt und im Falle von Z = –O–B die Schutzgruppe mit geeigneten Agentien entfernt oder

B) Aldehyde der allgemeinen Formel (II) in welcher $R^1$, $R^2$, A und X die in den Ansprüchen 1 bis 3 angegebene Bedeutung haben mit Ketonen der allgemeinen Formel (V)

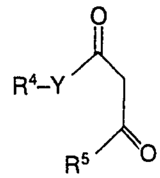

(V)

in welcher

$R^4$, $R^5$ die in den Ansprüchen 1 bis 3 angegebene Bedeutung haben und

mit Enaminen der allgemeinen Formel (VI)

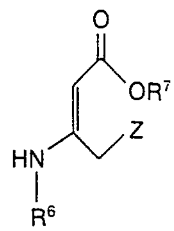

(VI)

in welcher

$R^6$, $R^7$ und Z die in den Ansprüchen 1 bis 3 bzw. die oben angegebene Bedeutung haben,

gegebenenfalls in Gegenwart inerter organischer Lösungsmittel bei Temperaturen zwischen 20 und 150 °C umsetzt und im Falle von Z = O–B, worin B die oben angegebene Bedeutung hat, anschliessend die Schutzgruppe mit geeigneten Agentien entfernt oder

C) Ylidenverbindungen der allgemeinen Formel (VII)

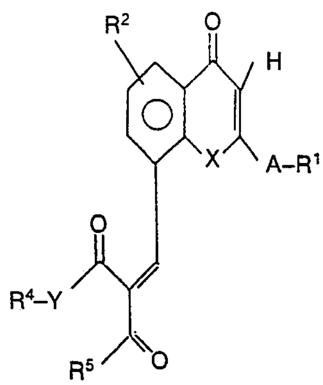

(VII)

in welcher

$R^1$, $R^2$, $R^4$, $R^5$, A, X und Y die in den Ansprüchen 1 bis 3 angegebene Bedeutung haben,

mit Enaminen der allgemeinen Formel (VI) in welcher $R^6$, $R^7$ und Z die in den Ansprüchen 1 bis 3 angegebene Bedeutung haben, gegebenenfalls in Gegenwart inerter organischer Lösungsmittel bei Temperaturen zwischen 20 und 150 °C umsetzt und im Falle von Z = O–B, worin B die oben angegebene Bedeutung hat, die Schutzgruppe anschliessend mit geeigneten Agentien abspaltet oder

D) Aldehyde der allgemeinen Formel (II) in welcher $R^1$, $R^2$, A und X die in den Ansprüchen 1–3

angegebene Bedeutung haben mit Ketonen der allgemeinen Formel (V) in welcher $R^4$ und $R^5$ die in den Ansprüchen 1 bis 3 angegebene Bedeutung haben und mit Tetronsäureamiden der allgemeinen Formel (VIII)

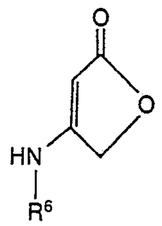

(VIII)

in der
$R^6$ die in den Ansprüchen 1 bis 3 angegebene Bedeutung hat,
gegebenenfalls in Gegenwart inerter organischer Lösungsmittel bei Temperaturen zwischen 20° und 150 °C umsetzt
oder
E) Benzylidenverbindungen der allgemeinen Formel (IX)

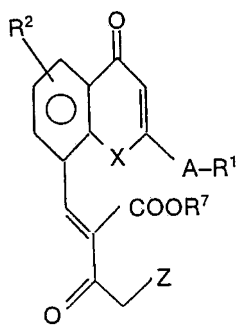

(IX)

in welcher

$R^1$, $R^2$, $R^7$, A, X und Z die in den Ansprüchen 1 bis 3 bzw. oben angegebene Bedeutung haben mit Enaminen der allgemeinen Formel (III), in welcher $R^4$, $R^5$, $R^6$ und Y die in den Ansprüchen 1 bis 3 angegebene Bedeutung haben, gegebenenfalls in inerten organischen Lösungsmitteln bei Temperaturen von 20 °C bis 150 °C umsetzt und im Falle von Z = O–B, worin B die oben angegebene Bedeutung hat, die Schutzgruppe mit geeigneten Agentien abspaltet.

6. Arzneimittel enthaltend mindestens eine Verbindung der Formel (I) gemäss Ansprüche 1 bis 2.

7. Verfahren zur Herstellung von Arzneimitteln, dadurch gekennzeichnet, dass man Verbindungen der Formel (I) gemäss Ansprüche 1 bis 3 gegebenenfalls unter Verwendung von üblichen Hilfs- und Trägerstoffen in eine geeignete Applikationsform überführt.

8. Verwendung der Verbindungen gemäss Ansprüche 1 bis 2 bei der Herstellung von Arzneimitteln zur Verbesserung der Herzkontraktilität, zur Erhöhung des Blutdruckes, zur Senkung von Blutzucker, zur Abschwellung von Schleimhäuten, zur Beeinflussung des Salz- und/oder Flüssigkeitshaushaltes.

**Claims**

1. Dihydropyridines of the general formula (I)

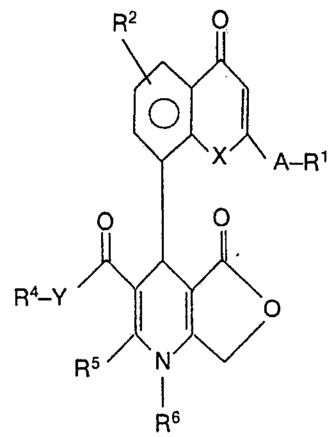

(I)

in which
$R^1$ represents hydrogen, a straight-chain, cyclic or branched aliphatic hydrocarbon with 1 to 8 C atoms, a carboxylic acid alkyl ester with 1 to 8 C atoms, in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl, it being possible for the aryl and heteroaryl radicals mentioned optionally to contain one to 5 identical or different substituents from the group comprising fluorine, chlorine, bromine, iodine, $C_1$–$C_8$-alkyl, $C_1$–$C_8$-alkylthio, $C_1$–$C_8$-alkylsulphinyl, cyano, hydroxyl, nitro, mono- or poly-fluoro-$C_1$–$C_4$-alkyl, mono- or polyfluoroalkoxy with 1 to 4 C atoms, mono- or polyfluoroalkylthio with 1 to 4 C atoms, amino, mono-alkylamino with 1 to 5 C atoms and dialkylamino with in each case 1 to 5 C atoms,
$R^2$ represents one to three halogen atoms or hydrogen and
$R^4$ represents a straight-chain, branched or cyclic saturated or unsaturated hydrocarbon radical with 1 to 18 C atoms, which is optionally substituted by $C_1$–$C_8$-alkoxy, $C_1$–$C_8$-alkylthio, $C_1$–$C_8$-alkylsulphinyl, trialkylsilyl with in each case 1 to 5 C atoms, Cl, Br, I, F, cyano, hydroxyl, amino, alkylamino with 1 to 5 C atoms, dialkylamino with in each case 1 to 5 C atoms, morpholinyl, piperidyl, piperazinyl, nitro, nitrate, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl or quinazolyl, it being possible for the aromatics or heteroaromatics mentioned optionally to be substituted by 1 to 3 identical or different substituents from the group comprising F, Cl, Br, $C_1$–$C_5$-alkyl, $C_1$–$C_5$-alkoxy, $C_1$–$C_4$-alkylthio and $C_1$–$C_4$-alkylsulphinyl,
$R^5$ represents hydrogen or a straight-chain, branched or cyclic, saturated or unsaturated aliphatic $C_1$–$C_8$-alkyl radical, which optionally contains one or two identical or different hetero chain members as the group comprising O, CO, S or N-alkyl with 1 to 6 C atoms, and is optionally substituted by halogen, nitro, cyano, hydroxyl, amino or mono-$C_1$–$C_5$-alkylamino or dialkylamino with in each case 1 to 5 C atoms,

$R^6$ represents hydrogen or a straight-chain or branched $C_1$–$C_{20}$-alkyl radical, which is optionally substituted by $C_1$–$C_{10}$-alkoxy, halogen or morpholino,

A represents a single bond or a $C_1$–$C_{20}$-alkylene chain, which chain can optionally be interrupted by O or S, with the condition that when A represents a single bond the radical $R^1$ cannot be hydrogen,

X represents O or S and

Y represents a single bond, O, S, –NH– or –N-alkyl with 1 to 8 C atoms,

in the form of isomers, isomer mixtures, racemates and optical antipodes, and their pharmaceutically acceptable salts.

2. Compounds according to Claim 1, in which

$R^1$ represents hydrogen, a straight-chain, cyclic or branched aliphatic hydrocarbon radical with 1 to 7 C atoms, a carboxylic acid alkyl ester with 1 to 6 C atoms in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl, benzimidazolyl or quinazolyl, it being possible for the aryl and heteroaryl radicals mentioned optionally to contain one to 4 identical or different substituents from the group comprising fluorine, chlorine, bromine, $C_1$–$C_6$-alkyl, $C_1$–$C_6$-alkylthio, $C_1$–$C_6$-alkylsulphinyl, cyano, hydroxyl, nitro, mono- or poly-fluoroalkyl with 1 to 3 C atoms, mono- or poly-fluoroalkoxy with 1 to 3 C atoms, amino, monoalkylamino with 1 to 4 C atoms and dialkylamino with in each case 1 to 4 C atoms,

$R^2$ represents hydrogen or one to three fluorine atoms,

$R^4$ represents a straight-chain, branched or cyclic, saturated or unsaturated hydrocarbon radical with 1 to 12 C atoms, which is optionally substituted by $C_1$–$C_6$-alkoxy, $C_1$–$C_6$-alkylthio, $C_1$–$C_6$-alkylsulphinyl, trialkylsilyl with in each case 1 to 3 C atoms, Cl, Br, F, cyano, hydroxyl, amino, alkylamino with 1 to 4 C atoms, dialkylamino with in each case 1 to 4 C atoms, morpholinyl, piperidyl, piperazinyl, nitro, nitrate, phenyl, naphthyl, thienyl, furyl, pyrryl, imidazolyl, oxazolyl, thiazolyl, pyridyl, pyrimidyl, pyrazinyl or indolyl, it being possible for the aromatics or heteroaromatics mentioned optionally to be substituted by 1 to 3 identical or different substituents from the group comprising F, Cl, Br, $C_1$–$C_4$-alkyl, $C_1$–$C_4$-alkoxy, $C_1$–$C_3$-alkylthio and $C_1$–$C_3$-alkylsulphinyl,

$R^5$ represents hydrogen or a straight-chain, branched or cyclic, saturated or unsaturated aliphatic alkyl radical with 1 to 6 C atoms, which optionally contains one or two identical or different hetero chain members from the group comprising O, CO, S or N-alkyl with 1 to 4 C atoms, and is optionally substituted by halogen, nitro, cyano, hydroxyl, amino, mono-$C_1$–$C_4$-alkylamino or dialkylamino with in each case 1 to 4 C atoms,

$R^6$ represents hydrogen or a straight-chain or branched $C_1$–$C_{12}$-alkyl radical, which is optionally substituted by $C_1$–$C_6$-alkoxy, halogen or morpholino,

A represents a single bond or a $C_1$–$C_{16}$-alkylene chain, which chain can optionally be interrupted by O or S, with the condition that when A represents a single bond the radical $R^1$ cannot be hydrogen,

X represents O or S and

Y represents a single bond, O, S, –NH– or –N(alkyl)- with 1 to 4 C atoms.

3. Compounds according to Claims 1 to 2 for combating diseases.

4. Compounds according to Claims 1 to 2 for improving heart contractility, as antihypotonic agents, for lowering the blood sugar level, for detumescing mucous membranes and for influencing the salt and/or liquid balance.

5. Process for the preparation of dihydropyridines of the general formula (I)

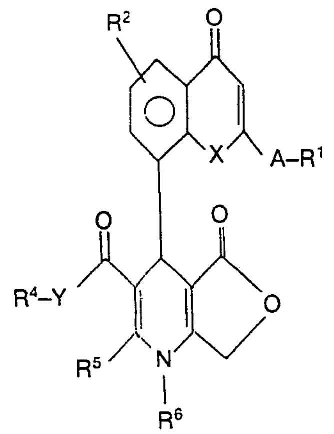

(I)

in which

$R^1$ represents hydrogen, a straight-chain, cyclic or branched aliphatic hydrocarbon with 1 to 8 C atoms, a carboxylic acid alkyl ester with 1 to 8 C atoms, in the alkyl chain, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, isoquinolyl, indolyl, benzimidazolyl, quinazolyl or quinoxalyl, it being possible for the aryl and heteroaryl radicals mentioned optionally to contain one to 5 identical or different substituents from the group comprising fluorine, chlorine, bromine, iodine, $C_1$–$C_8$-alkyl, $C_1$–$C_8$-alkylthio, $C_1$–$C_8$-alkylsulphinyl, cyano, hydroxyl, nitro, mono- or poly-fluoro-$C_1$–$C_4$-alkyl, mono- or polyfluoroalkoxy with 1 to 4 C atoms, mono- or polyfluoroalkylthio with 1 to 4 C atoms, amino, monoalkylamino with 1 to 5 C atoms and dialkylamino with in each case 1 to 5 C atoms,

$R^2$ represents one to three halogen atoms or hydrogen and

$R^4$ represents a straight-chain, branched or cyclic saturated or unsaturated hydrocarbon radical with 1 to 18 C atoms, which is optionally substituted by $C_1$–$C_8$-alkoxy, $C_1$–$C_8$-alkylthio, $C_1$–$C_8$-alkylsulphinyl, trialkylsilyl with in each case 1 to 5 C atoms, Cl, Br, I, F, cyano, hydroxyl, amino, alkylamino with 1 to 5 C atoms, dialkylamino with in each case 1 to 5 C atoms, morpholinyl, piperidyl, piperazinyl, nitro, nitrate, phenyl, naphthyl, thienyl, furyl, pyrryl, pyrazolyl, imidazolyl, oxazolyl, isoxazolyl, thiazolyl, pyridyl, pyridazinyl, pyrimidyl, pyrazinyl, quinolyl, indolyl or quinazolyl, it being possible for the aromatics or heteroaromatics mentioned optionally to be substituted by 1 to 3 identical or different substituents from the group comprising F, Cl, Br, $C_1$–$C_5$-alkyl, $C_1$–$C_5$-alkoxy, $C_1$–$C_4$-alkylthio and $C_1$–$C_4$-alkylsulphinyl,

$R^5$ represents hydrogen or a straight-chain, branched or cyclic, saturated or unsaturated aliphatic $C_1$–$C_8$-alkyl radical, which optionally contains one or two identical or different hetero chain members as the group comprising O, CO, S or N-alkyl with 1 to 6 C atoms, and is optionally substituted by halogen, nitro, cyano, hydroxyl, amino or mono-$C_1$–$C_5$-alkylamino or dialkylamino with in each case 1 to 5 C atoms,

$R^6$ represents hydrogen or a straight-chain or branched $C_1$–$C_{20}$-alkyl radical, which is optionally substituted by $C_1$–$C_{10}$-alkoxy, halogen or morpholino,

A represents a single bond or a $C_1$–$C_{20}$-alkylene chain, which chain can optionally be interrupted by O or S, with the condition that when A represents a single bond the radical $R^1$ cannot be hydrogen,

X represents O or S and

Y represents a single bond, O, S, –NH– or –N-alkyl with 1 to 8 C atoms,

characterised in that

A) aldehydes of the general formula (II)

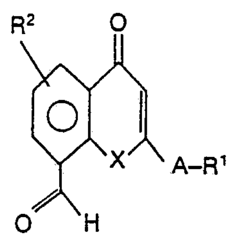

(II)

in which

$R^1$, $R^2$, A and X have the meaning given in Claims 1 to 2,

are reacted with enamines of the general formula (III)

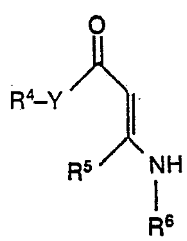

(III)

in which

$R^4$, $R^5$, $R^6$ and Y have the meaning given in Claims 1 to 2 and

ketones of the general formula (IV)

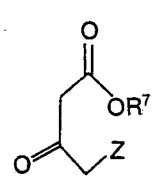

in which

$R^7$ represents an alkyl chain with 1 to 6 C atoms and

Z represents halogen or –O–B,

wherein

B represents an alcohol-protective group, such as

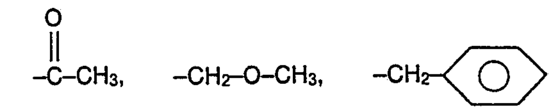

or $-Si(Alkyl)_3$

if appropriate in the presence of an inert organic solvent at temperatures between 20 and 150 °C, and, if Z = –O–B, the protective group is removed with suitable agents, or

B) aldehydes of the general formula (II) in which $R^1$, $R^2$, A and X have the meaning given in Claims 1 to 2, are reacted with ketones of the general formula (V)

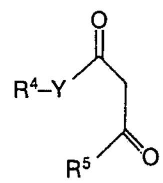

(V)

in which

$R^4$ and $R^5$ have the meaning given in Claims 1 to 2,

and enamines of the general formula (VI)

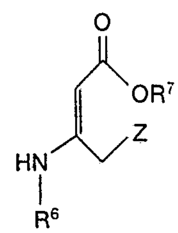

(VI)

in which

$R^6$, $R^7$ and Z have the meaning given in Claims 1 to 2 or the abovementioned meaning,

if appropriate in the presence of inert organic solvents at temperatures between 20 and 150 °C and, if Z = O–B, B having the abovementioned meaning, the protective group is then removed with suitable agents, or

C) ylidene compounds of the general formula (VII)

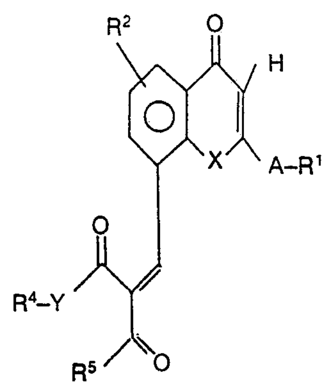

(VII)

in which

$R^1$, $R^2$, $R^4$, $R^5$, A, X and Y have the meaning given in Claims 1 to 2,

are reacted with enamines of the general formula (VI) in which $R^6$, $R^7$ and Z have the meaning given in Claims 1 to 2, if appropriate in the presence of

inert organic solvents at temperatures between 20 and 150 °C and, if Z = O–B, B having the abovementioned meaning, the protective group is then split off with suitable agents, or

D) aldehydes of the general formula (II) in which $R^1$, $R^2$, A and X have the meaning given in Claims 1–2, are reacted with ketones of the general formula (V) in which $R^4$ and $R^5$ have the meaning given in Claims 1 to 2, and with tetronic acid amides of the general formula (VIII)

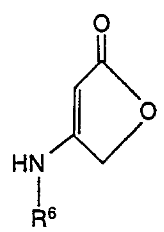

(VIII)

in which

$R^6$ has the meaning given in Claims 1 to 2, if appropriate in the presence of inert organic solvents at temperatures between 20° and 150 °C, or

E) benzylidene compounds of the general formula (IX)

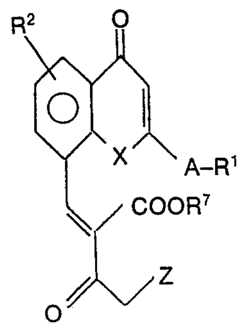

(IX)

in which

$R^1$, $R^2$, $R^7$, A, X and Z have the meaning given in Claims 1 to 2 or the abovementioned meaning, are reacted with enamines of the general formula (III) in which $R^4$, $R^5$, $R^6$ and Y have the meaning given in Claims 1 to 2, if appropriate in inert organic solvents at temperatures from 20 °C to 150 °C and, if Z = O–B, B having the abovementioned meaning, the protective group is split off with suitable agents.

6. Medicaments containing at least one compound of the formula (I) according to Claims 1 to 2.

7. Process for the preparation of medicaments, characterised in that compounds of the formula (I) according to Claims 1 to 2 are converted into a suitable administration form, if appropriate using the customary auxiliaries and excipients.

8. Use of the compounds according to Claims 1 to 2 in the preparation of medicaments for improving heart contractility, for increasing the blood pressure, for lowering the blood sugar level, for detumescing mucous membranes and for influencing the salt and/or liquid balance.

**Revendications**

1. Dihydropyridines de formule générale (I)

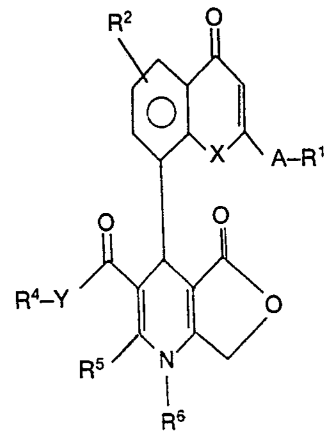

(I)

dans laquelle

$R^1$ représente de l'hydrogène, un hydrocarbure aliphatique à chaîne droite, cyclique ou ramifiée ayant 1 à 8 atomes de carbone, un ester alcoylé d'acide carboxylique ayant 1 à 8 atomes de carbone dans la chaîne alcoyle, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle, en l'occurrence les aryles ou hétéroaryles cités présentant éventuellement 1 à 5 substituants identiques ou différents du groupe fluor, chlore, brome, iode, alcoyle en $C_1$–$C_8$, alcoylthio en $C_1$–$C_8$, alcoylsulfinyle en $C_1$–$C_8$, cyano, hydroxy, nitro, alcoyle en $C_1$–$C_4$ mono- ou polyfluoré, mono- ou polyfluoralcoxy ayant 1 à 4 atomes de carbone, mono- ou polyfluoralcoylthio ayant 1 à 4 atomes de carbone, amino, monoalcoylamino ayant 1 à 5 atomes de carbone, dialcoylamino ayant chacun 1 à 5 atomes de carbone,

$R^2$ représente 1 à 3 atomes d'halogène ou de l'hydrogène et

$R^4$ représente un radical hydrocarboné à chaîne droite, ramifiée ou cyclique, saturé ou insaturé ayant 1 à 18 atomes de carbone, qui éventuellement est substitué par un alcoxy en $C_1$–$C_8$, alcoylthio en $C_1$–$C_8$, alcoylsulfinyle en $C_1$–$C_8$, trialcoylsilyle ayant chacun 1 à 5 atomes de carbone, Cl, Br, I, F, cyano, hydroxy, amino, alcoylamino ayant 1 à 5 atomes de carbone, dialcoylamino ayant chacun 1 à 5 atomes de carbone, morpholinyle, pipéridyle, pipérazinyle, nitro, nitrate, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle ou quinazolyle, en l'occurrence les composés aromatiques ou hétéroaromatiques cités pouvant éventuellement être substitués par 1 à 3 substituants identiques ou différents du groupe F, Cl, Br, alcoyle en $C_1$–$C_5$, alcoxy en $C_1$–$C_5$, alcoylthio en $C_1$–$C_4$, alcoylsulfinyle en $C_1$–$C_4$,

$R^5$ représente de l'hydrogène ou un radical alcoyle en $C_1$–$C_8$ à chaîne droite, ramifiée ou cyclique, saturé ou insaturé, aliphatique, qui contient éventuellement un ou deux hétérochaînons identiques ou différents comme le groupe O, CO, S ou N-alcoyle ayant 1 à 6 atomes de carbone et qui est éventuellement substitué par de l'halogène, nitro, cyano, hydroxy, amino, monoalcoylamino en $C_1$–$C_5$ ou dialcoylamino ayant chacun 1 à 5 atomes de carbone,

$R^6$ représente de l'hydrogène ou un radical alcoyle en $C_1$–$C_{20}$ à chaîne droite ou ramifiée qui est éventuellement substitué par un alcoxy en $C_1$–$C_{10}$, de l'halogène ou morpholino,

A représente une simple liaison, une chaîne alcoylène en $C_1$–$C_{20}$, la chaîne pouvant éventuellement être interrompue par O ou S, la condition étant que lorsque A représente une simple liaison, le radical $R^1$ ne peut pas être de l'hydrogène,

X représente O ou S et

Y représente une simple liaison, O, S, –NH– ou –N-alcoyle ayant 1 à 8 atomes de carbone, sous forme d'isomères, de mélanges d'isomères, de racémates et d'antipodes optiques et aussi de leurs sels pharmaceutiquement acceptables.

2. Composés suivant la revendication 1, dans lesquels

$R^1$ représente de l'hydrogène, un hydrocarbure aliphatique à chaîne droite ou ramifiée ayant 1 à 7 atomes de carbone, un ester alcoylé d'acide carboxylique ayant 1 à 6 atomes de carbone dans la chaîne alcoyle, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle, benzimidazolyle ou quinazolyle, en l'occurrence les aryles ou hétéroaryles présentant éventuellement 1 à 4 substituants identiques ou différents du groupe fluor, chlore, brome, alcoyle en $C_1$–$C_6$, alcoylthio en $C_1$–$C_6$, alcoylsulfinyle en $C_1$–$C_6$, cyano, hydroxy, nitro, mono- ou polyfluoralcoyle ayant 1 à 3 atomes de carbone, mono- ou polyfluoralcoxy ayant 1 à 3 atomes de carbone, amino, monoalcoylamino ayant 1 à 4 atomes de carbone, dialcoylamino ayant chacun 1 à 4 atomes de carbone,

$R^2$ représente de l'hydrogène ou 1 à 3 atomes de fluor,

$R^4$ représente un radical hydrocarboné à chaîne droite, ramifiée ou cyclique, saturé ou insaturé ayant 1 à 12 atomes de carbone, qui est éventuellement substitué par un alcoxy en $C_1$–$C_6$, un alcoylthio en $C_1$–$C_6$, un alcoylsulfinyle en $C_1$–$C_6$, un trialcoylsilyle ayant chacun 1 à 3 atomes de carbone, Cl, Br, F, cyano, hydroxy, amino, alcoylamino ayant 1 à 4 atomes de carbone dialcoylamino ayant chacun 1 à 4 atomes de carbone, morpholinyle, pipéridyle, pipérazinyle, nitro, nitrate, phényle, naphtyle, thiényle, furyle, pyryle, imidazolyle, oxazolyle, thiazolyle, pyridyle, pyrimidyle, pyrazinyle, indolyle, en l'occurrence les composés aromatiques ou hétéroaromatiques cités pouvant être éventuellement substitués par 1 à 3 substituants identiques ou différents du groupe F, Cl, Br, alcoyle en $C_1$–$C_4$, alcoxy en $C_1$–$C_4$, alcoylthio en $C_1$–$C_3$, alcoylsulfinyle en $C_1$–$C_3$,

$R^5$ représente de l'hydrogène ou un radical alcoyle aliphatique à chaîne droite, ramifiée ou cyclique saturé ou insaturé ayant 1 à 6 atomes de carbone, lequel éventuellement contient un ou deux hétérochaînons identiques ou différents du groupe O, CO, S ou N-alcoyle ayant 1 à 4 atomes de carbone et qui est éventuellement substitué par de l'halogène, nitro, cyano, hydroxy, amino, monalcoylamino en $C_1$–$C_4$ ou dialcoylamino ayant chacun 1 à 4 atomes de carbone,

$R^6$ représente de l'hydrogène ou un radical alcoyle en $C_1$–$C_{12}$ à chaîne droite ou ramifiée, qui est éventuellement substitué par un alcoxy en $C_1$–$C_6$, de l'halogène ou morpholino.

A représente une simple liaison, une chaîne alcoylène en $C_1$–$C_{16}$, en l'occurrence la chaîne pouvant éventuellement être interrompue par O ou S, la condition étant que lorsque A représente une simple liaison, le radical $R^1$ ne peut pas être de l'hydrogène,

X représente O ou S et

Y représente une simple liaison, O, S, –NH– ou –N(alcoyl) ayant 1 à 4 atomes de carbone.

3. Composés selon les revendications 1 à 2 pour combattre les maladies.

4. Composés selon les revendications 1 à 2 pour l'amélioration de la contractilité du cœur, comme antihypotonique, pour l'abaissement du sucre du sang, pour la non-érection (Abschwellung) des membranes, pour influencer le budget des sels et/ou des liquides.

5. Procédés de fabrication de dihydropyridines de formule générale (I)

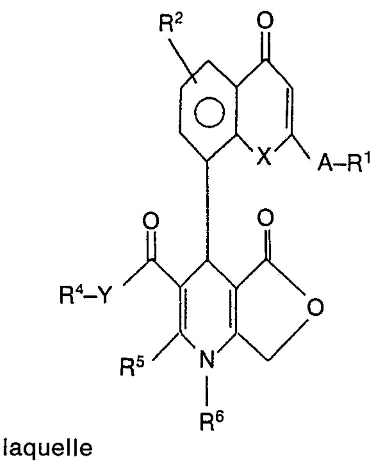

(I)

dans laquelle

$R^1$ représente de l'hydrogène, un hydrocarbure aliphatique à chaîne droite, cyclique ou ramifiée ayant 1 à 8 atomes de carbone, un ester alcoylé d'acide carboxylique ayant 1 à 8 atomes de carbone dans la chaîne alcoyle, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, isoquinolyle, indolyle, benzimidazolyle, quinazolyle ou quinoxalyle, en l'occurrence les aryles ou hétéroaryles cités présentant éventuellement un à cinq substituants identiques ou différents du groupe fluor, chlore, brome, iode, alcoyle en $C_1$–$C_8$, alcoylthio en $C_1$–$C_8$, alcoylsulfinyle en $C_1$–$C_8$, cyano, hydroxy, nitro, alcoyle en $C_1$–$C_4$ mono- ou polyfluoré, mono- ou polyfluoralcoxy ayant 1–4 atomes de carbone, mono- ou polyfluoralcoylthio ayant 1–4 atomes de carbone, amino, monoalcoylamino ayant 1 à 5 atomes de

carbone, dialcoylamino ayant chacun 1 à 5 atomes de carbone,

$R^2$ représente 1 à 3 atomes d'halogène ou de l'hydrogène et

$R^4$ représente un radical hydrocarboné à chaîne droite, ramifiée, cyclique, saturé ou insaturé ayant 1 à 18 atomes de carbone, qui est éventuellement substitué par un alcoxy en $C_1$–$C_8$, alcoylthio en $C_1$–$C_8$, alcoyle sulfinyle en $C_1$–$C_8$, trialcoylsilyle ayant chaque fois 1 à 5 atomes de carbone, Cl, Br, I, F, cyano, hydroxy, amino, alcoylamino ayant 1 à 5 atomes de carbone, dialcoylamino ayant chacun 1 à 5 atomes de carbone, morpholinyle, pipéridyle, pipérazinyle, nitro, nitrate, phényle, naphtyle, thiényle, furyle, pyrryle, pyrazolyle, imidazolyle, oxazolyle, isoxazolyle, thiazolyle, pyridyle, pyridazinyle, pyrimidyle, pyrazinyle, quinolyle, indolyle ou quinazolyle, en l'occurrence les composés aromatiques ou hétéroaromatiques cités pouvant éventuellement être substitués par 1 à 3 substituants identiques ou différents du groupe F, Cl, Br, alcoyle en $C_1$–$C_5$, alcoxy en $C_1$–$C_5$, alcoylthio en $C_1$–$C_4$, alcoylsulfinyle en $C_1$–$C_4$,

$R^5$ représente de l'hydrogène ou un radical alcoyle en $C_1$–$C_8$ aliphatique à chaîne droite, ramifiée ou cyclique, saturé ou insaturé, qui contient éventuellement 1 ou 2 hétérochaînons identiques ou différents sous forme du groupe O, CO, S ou N-alcoyle ayant 1 à 6 atomes de carbone et qui est éventuellement substitué par de l'halogène, nitro, cyano, hydroxy, amino, monoalcoylamino en $C_1$–$C_5$ ou dialcoylamino ayant chacun 1 à 5 atomes de carbone,

$R^6$ représente de l'hydrogène ou un radical alcoyle en $C_1$–$C_{20}$ à chaîne droite ou ramifiée, qui est éventuellement substitué par un alcoxy en $C_1$–$C_{10}$, de l'halogène ou morpholino,

A représente une simple liaison, une chaîne alcoylène en $C_1$–$C_{20}$, la chaîne pouvant être interrompue éventuellement par O ou S, la condition étant que lorsque A représente une simple liaison, le radical $R^1$ ne peut pas être de l'hydrogène,

X représente O ou S et

Y représente une simple liaison, O, S, –NH– ou –N-alcoyle ayant 1 à 8 atomes de carbone, caractérisé en ce qu'on fait réagir

A) des aldéhydes de formule générale (II)

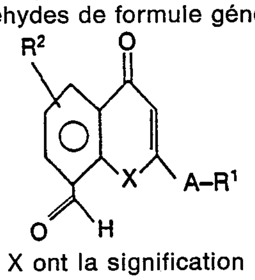

(II)

dans laquelle

$R^1$, $R^2$, A et X ont la signification indiquée aux revendications 1 à 3,

avec des énamines de formule générale (III)

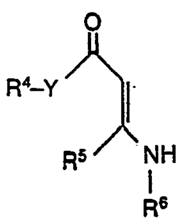

(III)

dans laquelle

$R^4$, $R^5$, $R^6$ et Y ont la signification indiquée aux revendications 1 à 3 et

avec des cétones de formule générale (IV)

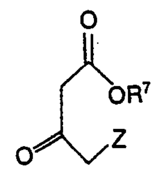

(IV),

dans laquelle

$R^7$ représente une chaîne alcoyle ayant 1 à 6 atomes de carbone et

Z représente de l'halogène ou –O–B, B étant un groupe protecteur d'alcool tel que

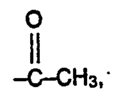 $-CH_2-O-CH_3$, 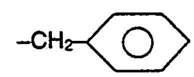

ou $-Si(alcoyl)_3$,

éventuellement en présence de solvants organiques inertes à des températures entre 20 et 150 °C et, dans le cas où Z = –O–B, on élimine le groupe protecteur avec des agents appropriés ou

B) on fait réagir des aldéhydes de formule générale (II) dans laquelle

$R^1$, $R^2$, A et X ont la signification indiquée aux revendications 1 à 3,

avec des cétones de formule générale (V)

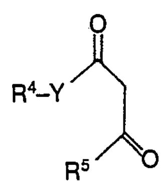

(V)

dans laquelle

$R^4$, $R^5$ ont la signification indiquée aux revendications 1 à 3 et

avec des énamines de formule générale (VI)

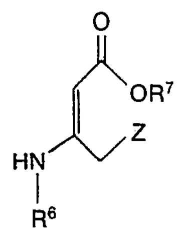

(VI)

dans laquelle

$R^6$, $R^7$ et Z ont la signification indiquée aux revendications 1 à 3,

éventuellement en présence de solvants organiques inertes à des températures entre 20 et 150 °C et en ce que dans le cas où Z = O–B, où B a la signification indiquée plus haut, on élimine ensuite le groupe protecteur avec des agents appropriés, ou

C) on fait réagir des composés ylidène de formule générale (VII)

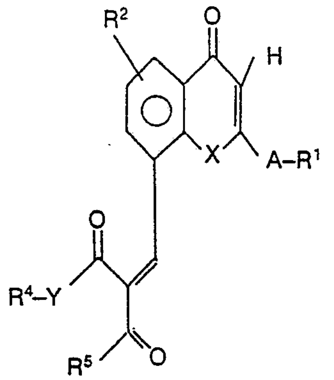

(VII)

dans laquelle

$R^1$, $R^2$, $R^4$, $R^5$, A, X et Y ont la signification indiquée aux revendications 1 à 3,

avec des énamines de formule générale (VI) dans laquelle

$R^6$, $R^7$ et Z ont la signification indiquée aux revendications 1 à 3,

éventuellement en présence de solvants organiques inertes à des températures entre 20 et 150 °C et en ce que dans le cas où Z=O–B, B ayant la signification indiquée plus haut, on clive ensuite le groupe protecteur avec des agents appropriés,

ou

D) on fait réagir des aldéhydes de formule générale (II) dans laquelle

$R^1$, $R^2$, A et X ont la signification indiquée aux revendications 1 à 3,

avec des cétones de formule générale (V) dans laquelle

$R^4$ et $R^5$ ont la signification indiquée aux revendications 1 à 3,

et avec des amides d'acide tétronique de formule générale (VIII)

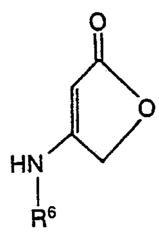

(VIII)

dans laquelle

$R^6$ a la signification indiquée aux revendications 1 à 3,

éventuellement en présence de solvants organiques inertes à des températures entre 20 et 150 °C,

ou

E) on fait réagir des composés de benzylidène de formule générale (IX)

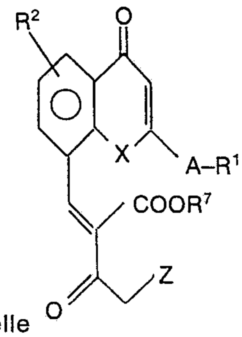

(IX)

dans laquelle

$R^1$, $R^2$, $R^7$, A et Z ont la signification indiquée plus haut aux revendications 1 à 3,

avec des énamines de formule générale (III), dans laquelle $R^4$, $R^5$, $R^6$, Y ont la signification indiquée aux revendications 1 à 3, éventuellement dans des solvants organiques inertes à des températures de 20 à 150 °C et en ce que dans le cas où Z = O–B, B ayant la signification indiquée plus haut, on clive le groupe protecteur avec des agents appropriés.

6. Médicaments contenant au moins un composé de formule (I) selon les revendications 1 à 2.

7. Procédé de fabrication de médicaments, caractérisé en ce qu'on convertit des composés de formule (I) selon les revendications 1 à 3, éventuellement avec utilisation de matières usuelles auxiliaires et de support, en une forme d'application appropriée.

8. Utilisation des composés selon les revendications 1 à 2 dans la fabrication de médicaments pour l'amélioration de la contractilité du cœur, pour l'élévation de la pression sanguine, pour l'abaissement du sucre dans le sang, pour la nonérection (Abschwellung) des membranes, pour influencer le budget des sels et/ou liquides.